Europäisches Patentamt

European Patent Office    (11) Publication number: **0 036 274**

Office européen des brevets    **B1**

(12)    EUROPEAN PATENT SPECIFICATION

(45) Date of publication of patent specification: **05.11.86**    (51) Int. Cl.⁴: **G 01 N 27/02,** G 01 N 33/48

(21) Application number: **81300928.9**

(22) Date of filing: **05.03.81**

(54) Methods of monitoring micro-organisms and media for culture.

(30) Priority: **08.03.80 JP 28581/80**
**08.03.80 JP 28582/80**

(43) Date of publication of application:
**23.09.81 Bulletin 81/38**

(45) Publication of the grant of the patent:
**05.11.86 Bulletin 86/45**

(84) Designated Contracting States:
**DE FR GB SE**

(56) References cited:
**DE-A-2 546 632**
**DE-A-2 747 033**
**FR-A-2 372 429**
**GB-A-1 299 363**
**GB-A-1 433 887**
**GB-A-2 029 026**
**US-A-4 009 078**

(73) Proprietor: **JAPAN TECTRON INSTRUMENTS CORPORATION**
**4-3-14, Nakamachi Koganei-shi**
**Tokyo (JP)**

(72) Inventor: **Fujioka, Hidehiko**
**268-402, Mogusa-Danchi 999, Mogusa Hino-shi**
**Tokyo (JP)**

(74) Representative: **Hartley, David et al**
**c/o Withers & Rogers 4 Dyer's Buildings Holborn**
**London, EC1N 2JT (GB)**

Courier Press, Leamington Spa, England.

**0 036 274**

## Description

Background of the invention
Field of the invention

The present invention relates to a method of monitoring micro-organisms. More particularly, the invention concerns methods of monitoring micro-organisms impedametrically, i.e. by measuring changes in electrical impedance of nutrient media upon which such micro-organisms are grown. The invention also concerns media which are used in carrying out the methods.

Description of the prior art:

Heretofore, identification of micro-organisms have usually relied on a biological character test in which discriminative identification of the micro-organism is primarily made on the basis of changes in color of an indicator as used. For example, in the case where organisms which are of interest and importance from the medical or clinical standpoint, a variety of indicators and reagents required for the microbiological character identification are added to nutrient agar media on which sampled organisms are inoculated to be subsequently cultured for 18 to 48 hours, wherein changes in color of the indicators as brought about by metabolic products discharged by the micro-organisms as the organisms achieve growth are visually observed to confirm the identity of the organisms.

The hitherto known methods of identifying the micro-organisms suffer many shortcomings. For example, a lot of time is required for a sampled organism to achieve growth sufficiently for affording the identification. As the consequence, in the case of medical diagnosis such long delay may force the physician to blindly initiate therapy without positive confirmation of the identity of the offending organisms, giving rise to a serious problem. Further, visual observation and discrimination of the cultured specimens require troublesome procedures of time-consumption and nevertheless provide sometimes the results of lessened reliability.

Recently, there have been issued reports on the methods of automatically and rapidly determining the organism by impedametrically monitoring the growth of organisms. Generally speaking, the impedametrical method is based on the fact that as the living organism grows in a liquid nutrient medium, they take in nutrients and discharge waste products, such process being generally known as the metabolic process, resulting in that electrical impedance of the medium undergoes corresponding variations. It has already been experimentally established that when suitable nutrient medium is employed, the rate of increase in the electrical impedance of a medium and the number of organisms correspond proportionately to the increase in the quantity of metabolic products produced as the organisms achieve the growth. Usually, the impedance tends to be decreased as the growth of organism proceeds.

In the case of the impedametrical method, which is a typical one of the hitherto known automatic and rapid organism identifying methods, the metabolic products of organism as cultured are detected as electrochemical changes of the whole nutrient media to thereby determine the rate of growth of the organism. However, because the metabolic process of organisms is generally of complex or complicated nature, the resultant impedance curve involves complexities in evaluation thereof. For this reason, the impedametrical method is not widely and generally adopted in the area of clinical bacteriology but restricted in application to the tests of antibiotic sensitivity and high speed measurement of the number of bacteria or organisms in urine. Although application of impedametric method to the identification of organisms or bacteria has been suggested, there have been yet no concrete proposal. At present, such application is considered to be difficult in practice. In reality, the impedametrical process is not made use of at all in the test concerning utilization of carbohydrates among the biological character tests.

An object of the invention is therefore to provide a method of impedametrically monitoring microorganisms which is immune to the drawbacks of the hitherto known method and which is capable of identifying the organisms with a sufficiently high speed for practical applications on the basis of particular variations in the electrical impedance of a nutrient or supporting medium.

In view of the above and other objects which will become more apparent as description proceeds, it is proposed according to an aspect of the invention that variation in electrical impedance of a medium having a predetermined composition which variation is brought about by the growth of organisms or bacteria and includes decrease and increase in impedance is measured and utilized through evaluation in examining the organisms cultured on the medium.

According to another aspect of the invention, it is proposed that the rates of decrease and increase in impedance for a unit time as well as time durations in which the impedance changes occur and the rate of decrease in impedance immediately before impedance is subsequently increased are detected to thereby allow identification of the microorganisms to be effected in a much facilitated manner.

In British Patent No. 1 433 887 there is described a method of detecting the growth of microorganisms. In the method of the Patent a nutrient medium is inoculated with a source of suspected microorganisms, incubated, and its conductivity measured at timed intervals. In that Patent it is further proposed to identify microorganisms by incubating them in a number of conductivity cells containing different media, some of which may contain antiserum, specific inhibitors, growth factors or antibiotics, the conductivity or time profiles produced by the growth of a particular organism on a number of different media resulting in a "fingerprint" characteristic of that organism.

2

The present invention turns on the discovery that, provided that both the concentrations of sodium and potassium salts, and the concentration of carbohydrate in the nutrient medium are kept within certain limits, and incubation is continued for a sufficient time, the impedance first diminishes, passes through a minimum, and then again increases, giving an impedance/time curve which is characteristic of the microorganism, and may be used to identify it.

The invention is defined in the claims appendant hereto and will be further described with reference to the accompanying drawings.

Figure 1 shows schematically an equivalent electric circuit of a medium in a cultivating and measuring cell;

Figure 2 is a block diagram showing an impedance measuring instrument used in carrying out the present invention;

Figure 3 shows a cultivating and measuring cell in a perspective view;

Figs. 4(A) to 4(F) graphically illustrate impedance variations brought about by enteric bacteria when glucose is used as nutrient;

Figs. 5(A) to 5(C) graphically illustrate impedance variations brought about by micro-organisms other than enteric bacteria when glucose is utilised as nutrient;

Figs. 6(A) to 6(D) graphically illustrate variation in impedance of media as a function of dissolved concentrations of disodium hydrogen phosphate;

Figs. 7(A) to 7(D) graphically illustrate impedance variations as a function of dissolved concentrations of sodium chloride;

Fig. 8 graphically illustrates variation in impedance of a medium constituted by a basal medium added with only electrolyte components;

Figs. 9(A) and 9(B) graphically illustrate variation in impedance as a function of dissolved concentrations of glucose;

Figs. 10(A) to 10(I) graphically illustrate variations in impedance when various carbohydrates are utilized as nutrient;

Figs. 11(A) to 11(I) graphically illustrate impedance variations when various aminoacids are utilized as nutrient; and

Fig. 12 is to graphically illustrate an example of evaluating a curve representing variation in impedance.

Detailed description of the preferred embodiments

In an experiment conducted by the present inventor concerning the growths of enteric bacteria with carbohydrates being utilized as nutrient, there was observed phenomena described below.

At first, a basal medium (Bacto-Peptone available from Difco Company) was added and mixed with a predetermined quantity of glucose to prepare a medium for use in identification. Since glucose or dextrose is a material which has a low electrical conductivity and exhibits a much higher impedance as compared with that of the components of the basal medium, increase and/or decrease in impedance which is directly ascribable to decrease of glucose due to consumption thereof by enteric bacteria could not be detected. In other words, no noticeable variation or change occurred in impedance of the supporting medium regardless of the growth of bacteria. Influence to impedance of the medium exerted by organic acids resulted from metabolic activity of bacteria could be observed only slightly. However, when a certain proper quantity of disodium hydrogen phosphate ($Na_2HPO_4$) is admixed to the basal medium mentioned above, inherent impedance of the medium was decreased. As the result, organic acids produced through bacterial metabolism from glucose could be detected with a significantly high level, making it possible to discriminate clearly difference in impedance variation or change due to the growth of bacteria. Similar phenomenon could be observed in the culture medium which was added with inorganic salts such as sodium chloride, as will be hereinafter described in more detail in conjunction with examples.

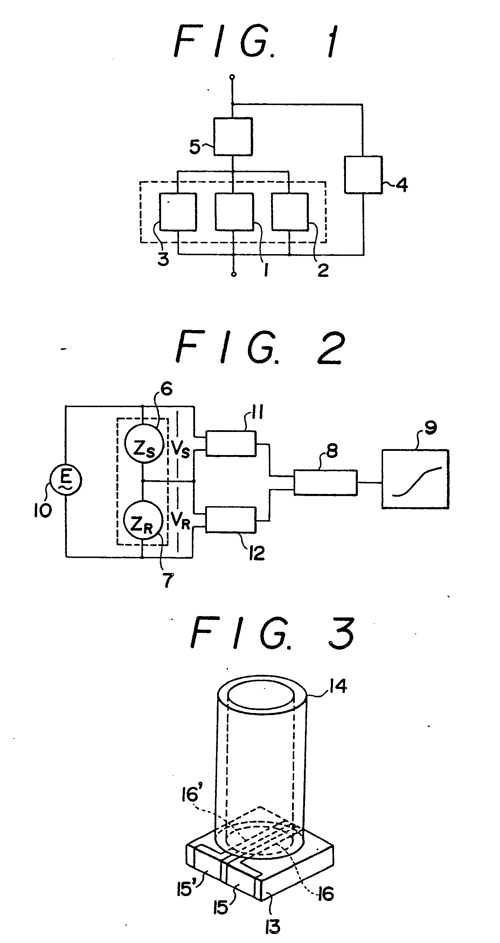

Now, referring to Fig. 1 which shows an electrically equivalent circuit of cultivation medium contained within a cultivating and measuring cell used in the experiments described just above, reference numeral 1 denotes an impedance component provided by the basal medium which contains scarcely sodium salts such as sodium chloride, disodium hydrogen phosphate or the like and electrolyte compounds such as potassium salts, calcium salts, magnesium salts or the like. Numeral 2 represents an impedance component provided by carbohydrates including glucose mannitol, and other carbohydrates. Numeral 3 represents an impedance component provided by inorganic salts including sodium chloride, disodium hydrogen phosphate, sodium dihydrogen phosphate and the like. The inorganic salt impedance component 3, the basal medium impedance component 1 and the carbohydrate impedance component 2 constitute medium impedance of the nutrient medium. Numeral 4 represents subtractive impedance provided by metabolic products including organic acids which exhibit lower impedance than the medium impedance mentioned above. Numeral 5 represents additive impedance provided by the metabolic products including organic acid, carbonic acid gases, hydrogen gas, water, ethanol and the like. It will be readily appreciated that the additive impedance component 5 and the subtractive impedance component 4 are ascribable to the progressive growth of bacteria.

The impedance components described above are composed of a pure resistance component for electrical conductivity and an electrostatic capacitance component of an aqueous electrolytic solution of

3

the supporting medium, wherein the resistance and the capacitance are connected in parallel with each other.

The supporting medium of the composition represented by the equivalent circuit diagram shown in Fig. 1 is inoculated with bacteria and incubated at a predetermined temperature. Then, growth of bacteria begins. As the growth proceeds, metabolic products are discharged. These metabolic products having respective electro-chemical constants cause impedance of the medium to be decreased and/or increased. Thus, when the impedance value of the medium is small before the growth of bacteria begins, the subtractive impedance component due to the metabolic products which is effective to decrease impedance of the medium as a whole is difficult to be detected. On the contrary, the additive impedance component which is ascribable to the products resulted from the metabolic activity of bacteria and effective to increase impedance of the medium as a whole becomes easier to be detected. On the other hand, in case the medium exhibits a high impedance value before the start of the multiplication of organisms, the subtractive impedance component ascribable to the metabolic products is easy to be detected, while the additive impedance component becomes difficult to be detected. In this conjunction, it should be mentioned that the variation in impedance will proceed only in the sense of decreasing in the case of the conventionally employed media.

By way of example, when coliform bacilli are incubated in aerobic state, the metabolic products of glucose supplied as nutrient will contain organic acids such as acetic acid, succinic acid, latic acid, or the like and additionally carbonic acid gas and water. Acetic acid and the like which are weak electrolytes exhibit relatively low electric conductivity and play a noticeable role in increasing impedance of the medium. The same applies true for carbonic acid gas and water. Further, carboxylic acids such as acetic acid and the like are ionized in the aqueous solution. When sodium ions ($Na^+$) are present, carboxylate ions ($RCOO^-$) will combine with sodium ions to form $RCOO^-Na^+$ ions, involving increase in the impedance value. In other words, when glucose or dextrose is made use of the metabolic process proceeds in the sense to increase impedance of the medium as a whole after a predetermined time has lapsed from the start of cultivation.

For the reasons described above, organic acids contained in the metabolic product can be detected as an increase in impedance with a high sensitivity and reliability by adding disodium hydrogen phosphate as well as strong electrolytes such as sodium, potassium salts or the like to the medium.

The inventor of the present application conducted similar experiments on several species of bacteria and obtained similar results. It is thus expected that substantially same or similar results can be obtained for other bacteria for which glucose can be utilized as nutrient. Further, under the same experimental conditions, different species of micro-organisms give rise to generation of impedance variation patterns each unique to each of organisms. Thus, it is possible to identify micro-organisms or bacteria on the basis of different impedance variation patterns.

Additionally, by virtue of the fact that degree of utilization of various carbohydrates including other sugars in addition to glucose can be detected as unique or peculiar variations in impedance, it is possible to carry out the hitherto known biological character identification test with the aid of the impedametrical process according to the invention.

Finally, decarboxylase test which has hitherto been adopted as one of the biological character tests that is important for identifying enteric bacteria can be carried out in a much facilitated and reliable manner by making use of the peculiar variation or change in impedance ascribable to the use of glucose as nutrient.

With the terms "decarboxylase test", it is intended to mean the test in which a medium added with proper quantities of grape sugar and aminoacid is inoculated with bacteria for incubation. Then, certain species of bacteria will, after acids have been produced through fermentation of glucose take in aminoacid and bring about decarboxylating reaction. Thus, change in color of an indicator as added allows bacteria to be identified.

However, it should be noted that any noticeable impedance variation will not make appearance if the media used heretofore in the decarboxylase test is employed. It has been found that difference in decarboxylation becomes significant only when weak electrolyte such as disodium hydrogen phosphate or the like salt is added to the hitherto used medium. When bacteria are cultured in the medium added with disodium hydrogen phosphate, acids are produced by bacteria from glucose after the growth has been initiated, involving decrease in the impedance value of the medium which is followed by an increase in the impedance value after a predetermined time has elapsed. However, when bacteria take in aminoacid after the acid production, the impedance value will not be increased but continuously being decreased. This phenomenon may be explained by the fact that when bacteria take in aminoacid, the latter is decomposed into carbonic acid gas and amines involving decrease in impedance by cancelling out action of $RCOO^-Na^+$ ions.

In this way, when a medium added with disodium hydrogen phosphate is used, the decarboxylation can be detected in a form of an impedance decrease pattern without fail. Other strong electrolyte salts than disodium hydrogen phosphate may be used to the same effect.

Similar results could be obtained from the same experiments performed on several species of bacteria. Thus, it is expected that substantially similar results can be obtained for other micro-organisms or bacteria which take in aminoacid. On the basis of the obtained results, identity of bacteria can be determined.

The culture medium according to the invention is constituted by a basal medium which includes hydrolyzate of proteins as a main component and added with at least carbohydrates and salts and/or aminoacid. The basal medium contains 2.5 to 10 g/l (gram per litre) of carbohydrates and 1.0 to 10 g/l of sodium and/or potassium salts dissolved in distilled water.

The basal medium functions as a nutrient source for the growth of micro-organism and contains as main components proteins such as peptones, triptones, meat extracts or the like which are used in aminoacid in bacteria. Carbohydrates are source of energy required for the growing of bacteria and comprises monosaccharides such as glucose, (dextrose), inositol, xylose, glycerols, arabinose, galactose, mannose, rhamnose, mannitol, sorbitol, adonitol, disaccharides such as lactose, sucrose, trehalose, melibiose, cellobiose, and polysaccharides such as raffinose, melezitose, dulcitol, amygdalin, salicin, fructose, (levulose).

Salts as added serve to catch metabolic products discharged by bacteria in a medium to thereby present peculiar changes in impedance of the medium, and may primarily include inorganic strong electrolytes such as sodium, potassium, magnesium, and calcium. Salt may be a mixture containing two or more components described above. There may be applications where vitamins are additionally added to the medium.

In the following, the invention will be described in more concrete in conjunction with examples.

(1) Types of bacteria and preparation of liquid phase inocula:

In the examples described hereinafter, experiments were made using nine types of enteric bacteria and other three types of bacteria as follows:

    A. Enteric bacteria
        a. *Escherichia coli* (coliform bacilli)
        b. *Serratia marcescens*
        c. *Klebsiella pneumonia* (Friedlaender's bacillus)
        d. *Klebsiella ozaenae*
        e. *Proteus vulgaris*
        f. *Enterobacter cloacae*
        g. *Proteus mirabilis*
        h. *Hafnia alvei*
        i. *Citrobacter freundi*
    B. Other types of bacteria
        j. *Staphylococcus aureus*
        k. *Streptococcus faecalis*
        l. *Pseudomonas aeruginosa*

Inocula in liquid phase were prepared through pure culture for 18 hours by using Heart Infusion Medium which is available from Eiken Chemistry Co. Ltd., in Japan. 10 µl of thus prepared inoculum was used for inoculation.

(2) Types of carbohydrates:

    Following eight types of carbohydrates were used.
        Glucose
        Mannitol
        Inositol
        Sorbitol
        Rhamnose
        Sucrose
        Melibiose
        Arabinose

(3) Types of aminoacids as employed:

    Following three types of aminoacids were employed.
        Alginine
        Lysine
        Ornithine

(4) Salts:

    Disodium hydrogen phosphate and sodium chloride were used.

(5) Preparation of media:

The basal medium as used was Bacto-Peptone (10 g/l) available from Difco Company. The media used in the exemplary experiments include the basal medium itself (medium ①), medium prepared by adding to the basal medium with 10 g/l of carbohydrate (medium ②), medium prepared by adding to the basal medium with 10 g/l of carbohydrate and disodium hydrogen phosphate (medium ③), medium prepared by adding to the basal medium with 10 g/l of carbohydrate and sodium chloride (medium ④), and medium

prepared by adding to the basal medium with 5 g/l of glucose, 2.5 g/l of disodium hydrogen phosphate and 10 g/l of aminoacid (medium ⑤). The media employed was 2 ml in volume. Different concentrations of disodium hydrogen phosphate and sodium chloride were employed.

(6) Principle of impedance measurement and instrument:

The tool used for measuring the impedance variations brought about by the growth of bacteria was an instrument which is commercially available from Japan Tectron Instruments Co. Ltd. in Japan under the trade name "Micro-organism Culture Automatic Measuring Apparatus" of Type Orga-6.

Fig. 2 shows the measuring apparatus Orga-6 described above in a schematic block diagram. A sample cell 6 and a reference cell 7 are directly connected in an A.C. excitation circuit. Voltages appearing across the sample cell 6 and the reference cell 7 are measured and the ratio of these voltages is arithmetically determined. The impedance ratio is determined from the above voltage ratio in terms of $ZSt/ZRt=VSt/VRt$ where $ZR$ and $VR$ represent impedance and voltage of the sample cell 6, respectively. Accordingly, change or variation in the impedance ratio at a time $t$ from the time of inoculation is given by $VSo/VRo-VSt/VRt$. Thus, the measured value is given by $(VSo/VRo-VSt/VRt)/(VSo/VRo)$ after having been normalized by a base value of $VSo/VRo$. The measured value is arithmetically processed in an arithmetic operation circuit 8, whereby the impedance change as determined is recorded by a recording apparatus 9. Reference numeral 10 denotes a power supply source, and 11 and 12 denote amplifier circuits.

(7) Cultivating and measuring cell:

A cell for cultivating and measuring bacteria as employed in the experimental examples is shown in Fig. 3. The cell comprises a platform 13 of about 20 mm square on which a cylindrical body 14 of about 40 mm in height is fixedly and integrally secured. A pair of electrodes 16 and 16' formed purely of gold are disposed on an inner bottom of the cylindrical body 14 with terminals 15 and 15' for the electrodes being provided on the platform 13. Impedance value of medium is measured between the electrodes 16 and 16'. The cell is made of hard glass so as to be re-usable after vapor sterilization at 121°C for 15 minutes.

The conditions for measuring impedance are as follows:

Source frequency: 1 kHz
Impedance Detection Sensitivity: 10% in full-scale
Measuring Period: 8 hours (H.) in full-scale or 16 hours (H.) in full-scale
Culture Temperature: 35°C
Preincubation Period: 30 minutes (min)

Now, examples of the invention will be described below.

Example 1

In this example, it has been experimentally ascertained how the change or variation in impedance which occurs in the medium as enteric bacteria grow by taking in glucose differs peculiarly in dependence on the types of media as well as the types of bacteria.

Six types of bacteria $a$ to $f$ enumerated herein above were used. Those bacteria underwent pure cultures in "Heart Infusion" media stated before for 18 hours, respectively. Liquid inocula each of 10 μl were inoculated in respective specimen cells described below.

Three types of the media ①, ② and ③ were prepared each in duplicate for each type of bacteria. More specifically, three specimen cells were used. A first cell was charged with 2 ml of medium ①, the second specimen or sample cell was charged with 2 ml of medium ② and the third was charged with 2 ml of medium ③. The media ② and ③ were added with 10 g/l of grape sugar. The medium ③ was further added with 2.5 g/l of disodium hydrogen phosphate.

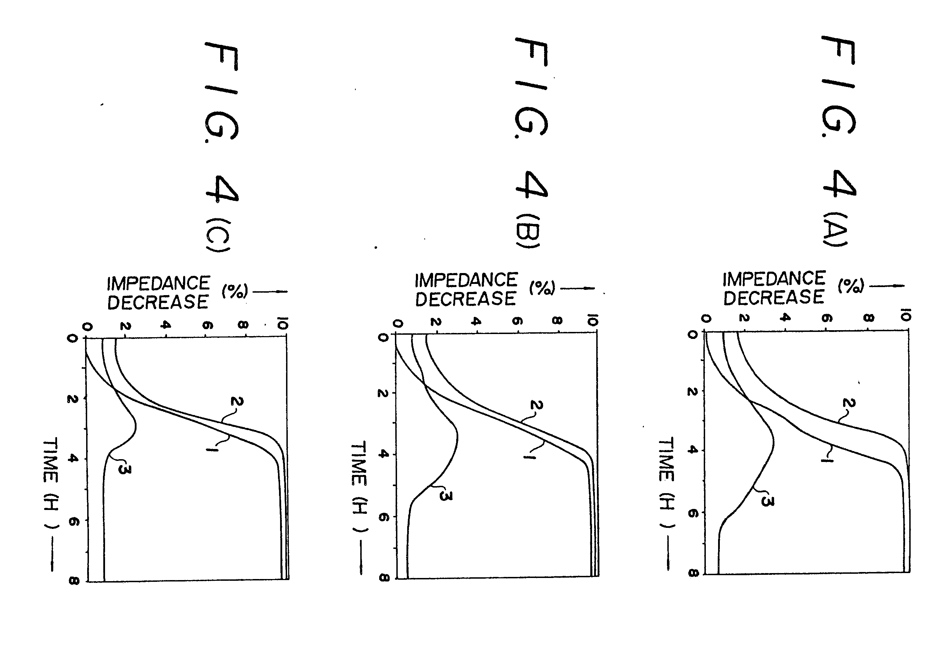

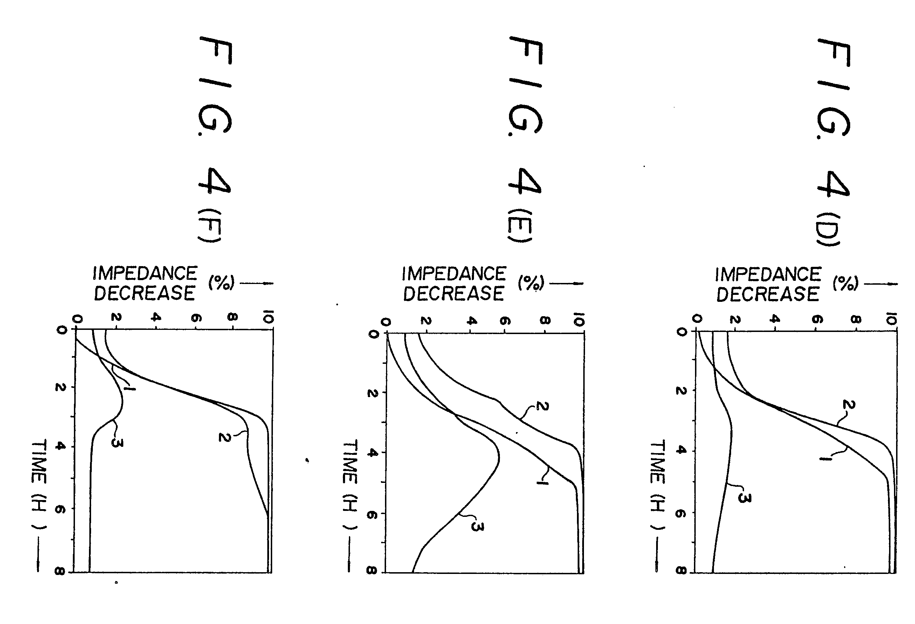

On the conditions described above, bacteria were cultivated and impedance changes of the media were measured by using the instrument Orga 6. The impedance changes as recorded are shown in Figs. 4(A) to 4(F). In the graphs shown in these figures, incubation period or time in hour is taken along the abscissa, while rate of impedance decrease is taken along the ordinate. Figs. 4(A) shows the results of impedance measurement for *Escherichia coli*, Fig. 4(B) is for *Serratia marcescens*, Fig. 4(C) is for *Klebsiella pneumonia*, Fig. 4(D) is for *Klebsiella ozaenae*, Fig. 4(E) is for *Proteus vulgaris*, and Fig. 4(F) is for *Enterobacter cloacae*. In these figures, attached reference numeral 1 represents the case where the medium ① is used, and numeral 2 represents the use of the medium ② while numeral 3 represents the use of the medium ③.

As will be appreciated from these graphs, in the case of the medium ② which is prepared by adding only 10 g/l of glucose to the basal medium, utilization of sugar does not appear in the form of appreciable impedance change but involves merely impedance decrease similar to that of the basal medium ①. In contrast, in the case of the medium ③ which has been prepared by adding 2.5 g/l of disodium hydrogen phosphate to the basal medium in addition to 10 g/l of glucose there make appearance very significant impedance changes for all the types of bacteria, each impedance change being peculiarly characteristic of each bacterium. This means that degree of utilization of glucose differs in dependence on the types of bacteria. In conclusion, it can be said that as bacteria grow, the impedance of each of the associated media is decreased in the initial phase in common to all the three types of media, and that after elapse of a predetermined time (two to five hours) increasing in impedance takes place only in the case of medium ③.

Example 2

Experiments similar to those mentioned above in combination with Example 1 were performed except that other than enteric bacteria were employed.

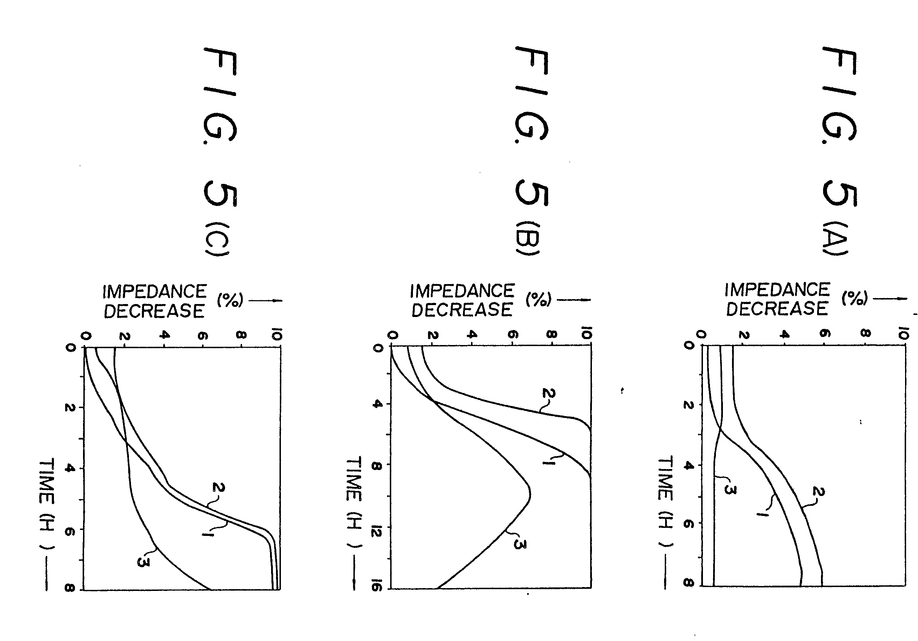

Three supporting media ①, ② and ③ were prepared in the quite same manner as in the case of Example 1 and inoculated, respectively, with bacteria of the types *j*, *k* and *l* enumerated hereinbefore. Impedance changes were measured and recorded in the same manner. The results as obtained are graphically illustrated in Figs. 5(A) to 5(C). More specifically, the graph shown in Fig. 5(A) represents the experimental results obtained for *Staphylococcus aureus* (bacterium *j*), the graph of Fig. 5(B) is for *Streptococcus faecalis* (bacterium *k*), and the graph of Fig. 5(C) is for *Pseudomonas aeruginosa* (bacterium *l*). Further, curves 1, 2 and 3 represent impedance change characteristics of the media ①, ② and ③, respectively.

It will be seen that, for the medium 3, *Staphylococcus aureus* and *Streptococcus faecalis* bring about significant impedance changes. However, *Pseudomonas aeruginosa* having no function of fermenting glucose for utilization thereof brings about no impedance change for any one of the media ①, ② and ③.

It will further be noted that, in the case of *Staphylococcus aureus*, the transition point from the decrease to the increase in impedance corresponds to a time lapse of about 2 hours, while the transition point in the case of *Streptococcus faecalis* occurs after time lapse of about 8 hours.

Example 3

In this Example, influences of the quantity of disodium hydrogen phosphate added to the basal medium (i.e. the dissolved concentration of disodium hydrogen phosphate) to the impedance change of the medium were demonstrated.

Four kinds of bacteria, i.e. *Escherichia coli, Klebsiella pneumonia, Proteus vulgaris* and *Staphylococcus aureus* were used in the present Example. Cultivation and inoculation were effected in the same manner as in the case of Example 1.

In the first place, starting from the basal medium containing 10 g/l of glucose, six types of media of different concentrations of disodium hydrogen phosphate in concentration series of 0 g/l, 0.25 g/l, 0.5 g/l, 1.0 g/l, 2.5 g/l and 5.0 g/l were prepared for each of *Klebsiella ozaenae* and *Staphylococcus aureus*, while eight types of media including 10 g/l and 20 g/l of disodium hydrogen phosphate in addition to the above concentration series were prepared for each of *Escherichia coli* and *Proteus vulgaris*. The media thus prepared were charged in respective specimen cells each by 2 ml.

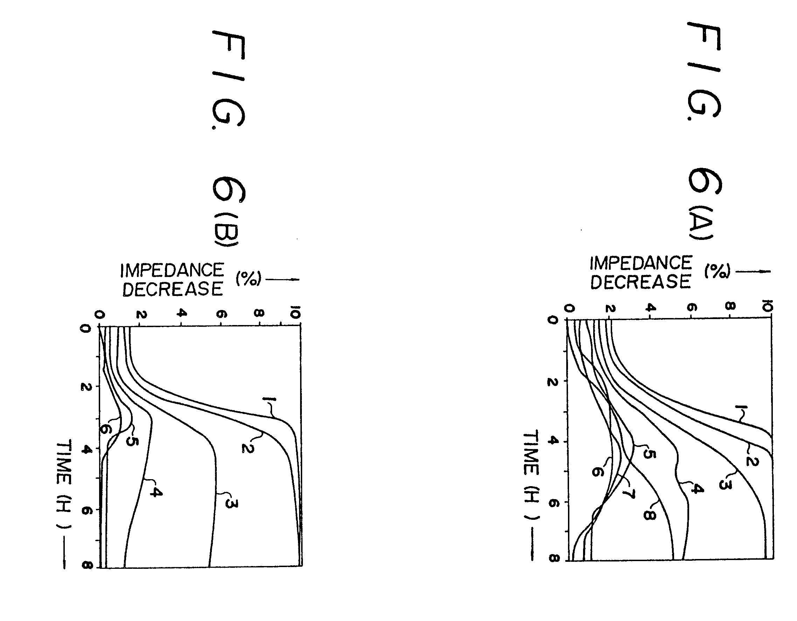

On the conditions mentioned above, cultivation and impedance measurement were performed with the aid of the instrument Orga 6. The results are graphically illustrated in Figs. 6(A) to 6(D), wherein Fig. 6(A) illustrates the impedance changes as a function of the culture period for cultivation of *Escherichia coli*, Fig. 6(B) shows corresponding graphs for *Klebsiella pneumonia*, Fig. 6(C) is for *Proteus vulgaris*, and Fig. 6(D) is for *Staphylococcus aureus*. In these figures curves 1, 2, 3, 4, 5, 6, 7 and 8 corresponds, respectively, to different concentrations of disodium hydrogen phosphate described above. More specifically, the curves 1 corresponds to 0 g/l of disodium hydrogen phosphate. Curve 2 corresponds to 0.25 g/l of disodium hydrogen phosphate. In a similar manner, the curves 3, 4, 5, 6, 7 and 8 correspond, respectively, to 0.5 g/l, 1 g/l, 2.5 g/l, 5 g/l, 10 g/l and 20 g/l of disodium hydrogen phosphate.

It will be noted that when the basal medium is added with only glucose, utilization thereof by bacteria does not bring about any appreciable change in impedance, which makes however significant appearance in the case where disodium hydrogen phosphate is added. Further, as the concentration of disodium hydrogen phosphate is increased, impedance tends to increase at a greater rate up to the concentration of disodium hydrogen phosphate of 5 g/l. Such tendency can be observed in cultures of all four types of bacteria. Further, it will be noted that influence of increase in the quantity of disodium hydrogen phosphate as added to the impedance change becomes insignificant when the concentration in concern is increased beyond 20 g/l. Thus, it is considered that the effective concentration of disodium hydrogen phosphate is in a range from 1.0 g/l to 10 g/l. More preferably, the concentration range from 2.5 g/l to 5 g/l makes more discriminative the difference in the impedance changes brought about by different types of bacteria. In the case of examples illustrated in Fig. 6(D), the curves 3, 4, 5 and 6 are flat, because rate of impedance change is positive.

Example 4

In this example, it is investigated how the quantity of sodium chloride added to the basal medium exerts influence to the impedance change of the medium.

To this end, four types of bacteria, that is, *Escherichia coli, Klebsiella pneumonia, Proteus vulgaris, Staphylococcus aureus* were used. The cultivation and inoculation were processed in the same manner as in the case of Example 1. Further, six dissolved concentrations were employed which were same as the first to the six concentrations in the series described above in conjunction with Example 3.

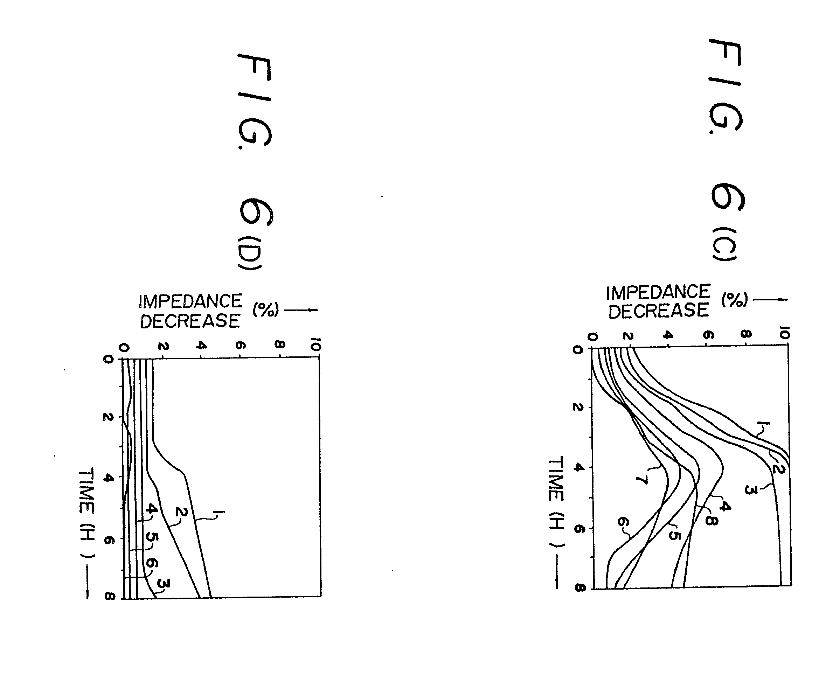

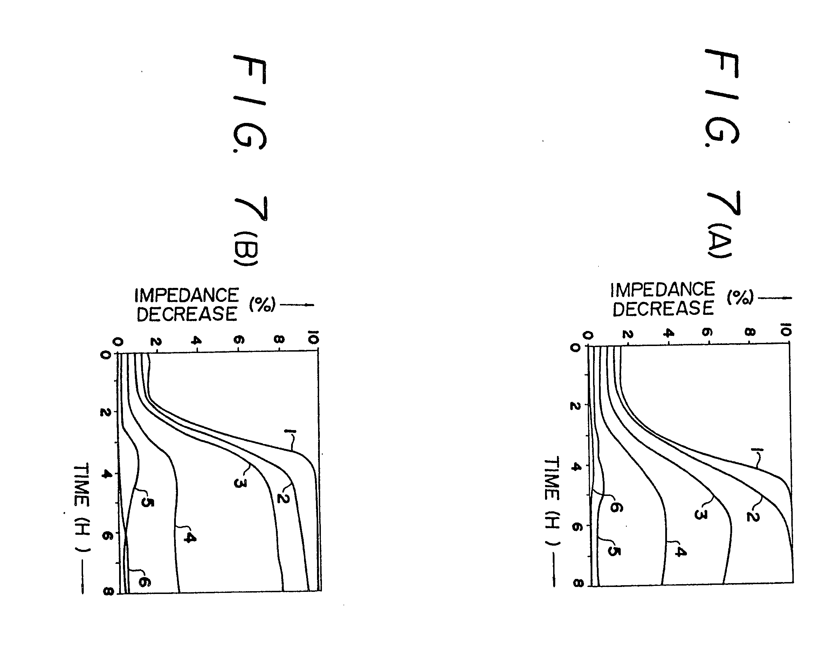

Figs. 7(A) to 7(D) graphically illustrates the results of measurements as obtained with the aid of the apparatus Orga 6. More specificlaly, Fig. 7(A) illustrates impedance changes brought about in the media by

7

*Escherichia coli*. By the same token, Fig. 7(B) is for *Klebsiella pneumonia*, Fig. 7(C) is for *Proteus vulgaris* and Fig. 7(D) is for *Staphylococcus aureus*. Further, curves 1 to 6 in each of these figures correspond to the different concentrations of sodium chloride as employed.

It will be seen that also in the present Example, impedance tends to be increased, as concentration of dissolved sodium chloride becomes higher. Thus, utilizations of glucose by different types of bacteria can be clearly discriminated. It is considered that the lower limit of the concentration threshold of sodium chloride is of the order of 1.0 g/l. The curves 4, 5 and 6 shown in Fig. 7(D) are flat, because the rate of impedance change is positive.

From the Examples 3 and 4, it will be understood that addition of strong electrolyte components such as disodium hydrogen phosphate and sodium chloride in a predetermined quantity range contributes to increase impedance of the medium. This may be explained by the fact that organic acids produced through metabolic activity of bacteria are weak electrolytes and impedance is increased rather than decreased after lapse of a predetermined time since initiation of the utilization or consumption of sugar by bacteria.

Example 5

In this example, impedance change was observed when only electrolyte component was added to a medium with a view to determining whether or not identification of bacteria is possible in the medium which contains no carbohydrates.

*Escherichia coli* was employed. Cultivation and inoculation were performed in the same manner as in the case of Example 1. Sodium chloride was used as an electrolyte with six different concentrations of dissolution in the same concentration series as in the case of Example 4.

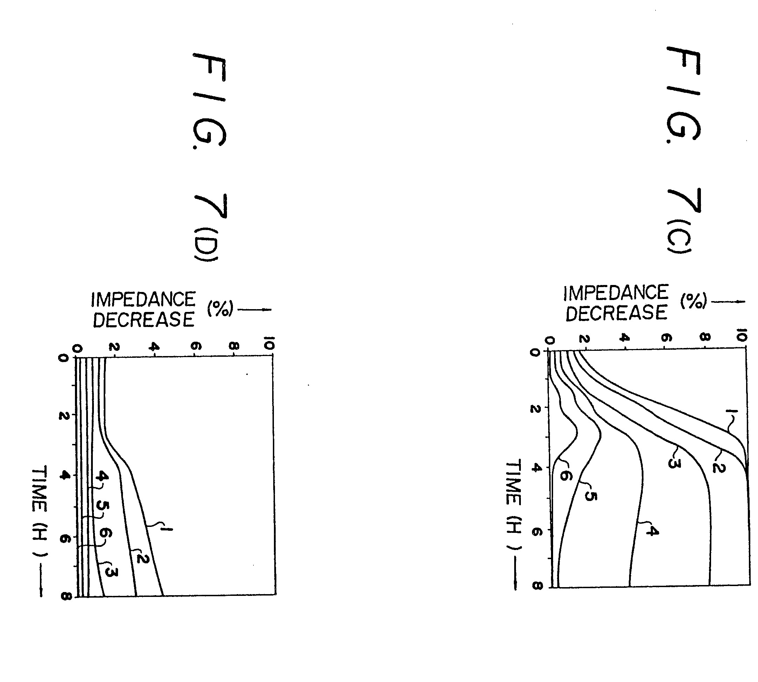

Fig. 8 shows the results of measurements obtained by using the instrument Orga 6. Curves 1, 2, 3, 4, 5 and 6 corresponds to the different concentrations of added sodium chloride of 0 g/l, 0.25 g/l, 0.5 g/l, 1.0 g/l, 2.5 g/l and 5.0 g/l, respectively, as in the case of Example 4.

It will be seen that any peculiar impedance changes of interest occur for all the six different types of media. The impedance tends to simply decrease as a function of time. This phenomenon may be explained by the fact that since no carbohydrates are contained, organic acid discharged through metabolic activity of the bacterium is of the negligible order. The same hold true for the case where disodium hydrogen phosphate is added.

Example 6

In the present example, the experiment is intended to determine influence of the concentration of dissolved carbohydrates exerted to impedance change of the medium.

Two types of bacteria, i.e. *Escherichia coli* and *Proteus vulgaris* were used. Cultivation and inoculation were carried out in the same manner as in the case of the Example 1. A reference or control medium was prepared by adding 5 g/l of disodium hydrogen phosphate to the basal medium containing 10 g/l of peptone. Further, specimen or sample media were prepared by adding to the reference or control medium glucose in 1 g/l, 2.5 g/l, 5 g/l, 10 g/l and 20 g/l, respectively.

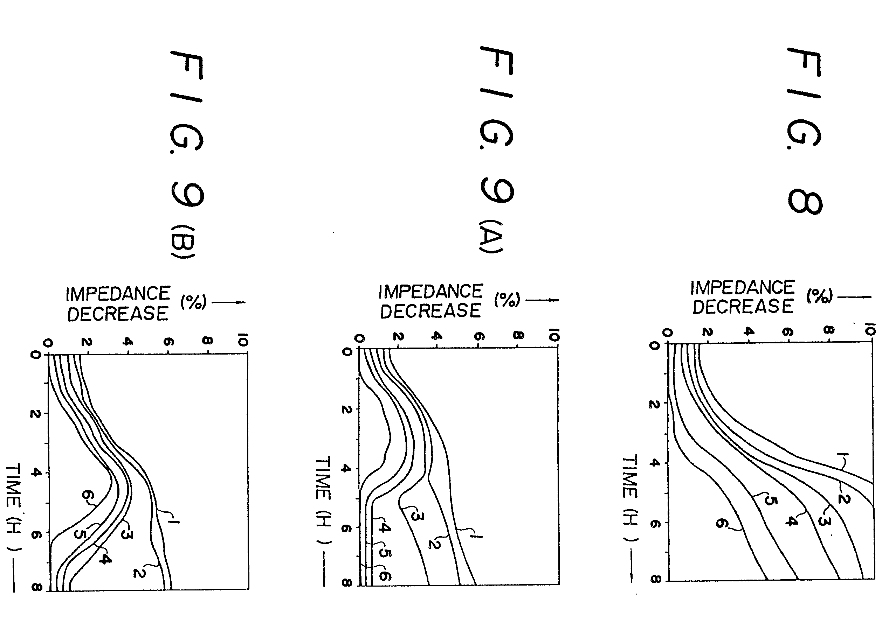

The results of measurements made for the two types of bacteria mentioned above by using these media in the apparatus Orga 6 are graphically illustrated in Figs. 9(A) and 9(B). The impedance changes brought about by *Escherichia coli* are represented by curves 1, 2, 3, 4, 5 and 6 for the six different concentrations of glucose mentioned above in Fig. 9(A), while Fig. 9(B) illustrates impedance changes of the different media upon which *Proteus vulgaris* was grown.

As can be seen from the graphic illustrations, when no glucose is added or when only a small quantity of glucose is added, no significant change in the sense of increase in impedance makes appearance. However, when the quantity of added glucose exceeds 2.5 g/l, there occur distinctly peculiar changes in impedance.

It will thus be understood that 2.5 g/l or more of carbohydrates should be added in order to produce peculiar impedance change of interest in the medium. The same hold true for other carbohydrates such as mannitol, inositol, sorbitol, rhamnose, sucrose, melibiose, arabinose and the like.

Example 7

Impedance changes brought about by consumption of various carbohydrates by enteric bacteria were experimentally determined to ascertain that degree of utilization or consumption becomes different in dependence on the types or kinds of carbohydrates contained in the media, whereby impedance changes characteristic of different bacteria are also rendered different.

Carbohydrates as employed includes glucose, mannitol, inositol, sorbitol, rhamnose, sucrose, melibiose, and arabinose. Nine types of bacteria were used. They are *Escherichia coli, Serratia marcescens, Klebsiella pneumonia, Klebsiella ozaenae, Proteus vulgaris, Enterobacter cloacae, Proteus mirabilis, Hafnia alveci* and *Citrobacter freundi*. Eight types of sample cells each of 2 ml were prepared each by adding to the basal medium with one of carbohydrates mentioned above together with 5 g/l of disodium hydrogen phosphate. Liquid inocula each of 10 μl to be inoculated in the sampels were prepared by the same procedures as in the case of the Example 1.

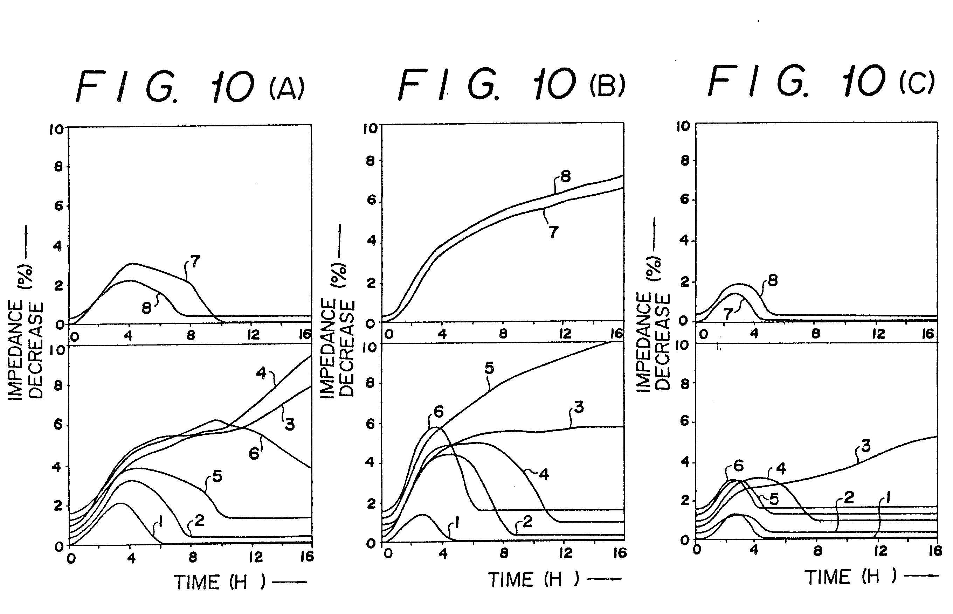

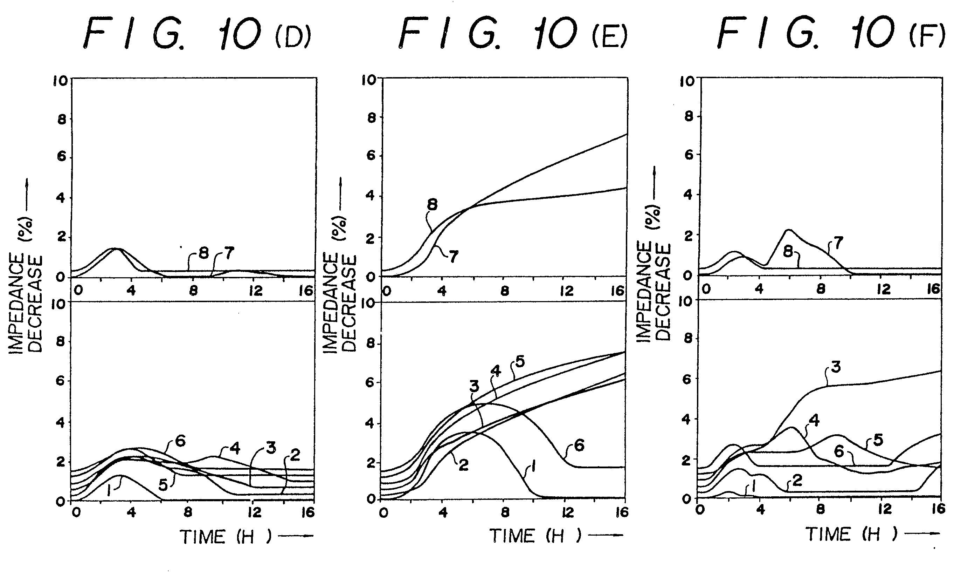

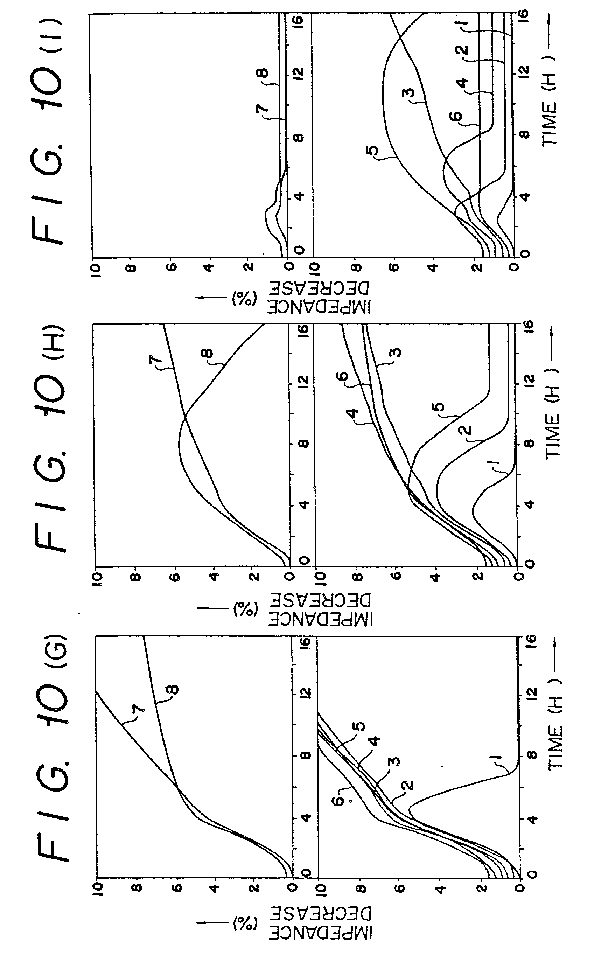

Figs. 10(A) to 10(I) graphically illustrate results of the measurements made by using the apparatus Orga 6. Fig. 10(A) represents the measurement results for *Escherichia coli*, Fig. 10(B) is for *Serratia*

*marcescens*, Fig. 10(C) is for *Klebsiella pneumonia*, Fig. 10(D) is for *Klebsiella ozaenae*, Fig. 10(E) is *Proteus vulgaris*, Fig. 10(F) is for *Enterobacter cloacae*, Fig. 10(G) is for *Proteus mirabilis*, Fig. 10(H) is for *Hafnia alvei*, and Fig. 10(I) is for *Citrobacter freundi*. Further, impedance characteristic curves 1, 2, 3, 4, 5, 6, 7 and 8 correspond to the uses of glucose, mannitol, inositol, sorbitol, rhamnose, sucrose, melibiose and arabinose, respectively.

It has been ascertained from this experiment that degree of utilization of carbohydrate by the various bacteria in formation of acids differs in dependence on the types of carbohydrates as used. More specifically, in the case where the impedance value continues to decrease, carbohydrate is not utilized. When impedance increases on the way, this means that carbohydrate is utilized. Further, differences in utilization of carbohydrate can be distinctly discriminated on the basis of profiles of the impedance characteristic curves. Thus, the results of measurement can be made use of as the criterion for identifying bacteria. In other words, it is decided that bacterium is negative for carbohydrate for which the impedance value of the associated medium continues to decrease. On the other hand, bacteria are decided to be posite for carbohydrate for which the impedance value of the associated media turn to increase on the way. Such decision is made for each of the eight types of carbohydrates and utilized for identifying bacteria through comparison with the known data. In Table 1 recited below, utilizations of carbohydrates by various bacteria are listed. In this table, plus sign (+) means that bacteria are positive, while minus sign (−) means that bacteria are negative. When the results of the present Example were compared with those obtained through the same procedure by using a commercially available bacterium identifying kit "API 20E" (manufactured by API company), it was confirmed that both results essentially coincide with each other.

9

TABLE 1

Evaluation of utilization of carbohydrates by bacteria

| Carbohydrate Bacteria | Glucose | Mannitol | Inositol | Sorbitol | Rhamnose | Sucrose | Melibiose | Arabinose |
|---|---|---|---|---|---|---|---|---|
| Escherichia coli | + | + | − | − | + | + | + | + |
| Serratia marcescens | + | + | − | + | − | + | − | − |
| Klebsiella pneumonia | + | + | − | + | + | + | + | + |
| Klebsiella ozaenae | + | + | + | + | + | + | + | + |
| Proteus vulgaris | + | − | − | − | − | + | − | − |
| Enterobacter cloacae | + | + | − | + | + | + | + | + |
| Proteus mirabilis | + | − | − | − | − | − | − | − |
| Hafnia alvei | + | + | − | − | + | − | − | + |
| Citrobacter freundi | + | + | − | + | + | + | + | + |

Example 8

In the present Example, decarboxylation tests were carried out in which peculiar impedance changes as brought about by utilization of carbohydrate by bacteria (i.e. fermentation and utilization of glucose) are made use of to ascertain that the impedance characteristic curves differ in dependence on the types of amino acids added to media.

Aminoacids employed in this example includes alginine, lysine and ornithine. Nine types of bacteria were used. They are *Escherichia coli*, *Serratia marcescens*, *Klebsiella pneumonia*, *Klebsiella ozaenae*, *Proteus vulgaris*, *Enterobacter cloacae*, *Proteus mirabilis*, *Hafnia alvei* and *Citrobacter freundi*. The media were prepared by adding 5 g/l of grape sugar and 2.5 g/l of disodium hydrogen phosphate to 10 g/l of the basal medium and further added with each of aminoacids described above in 10 g/l. Thus, there were used three types of sample cells each of 2 ml for each of the nine types of bacteria enumerated above. The liquid inocula each of 10 l to be grown on these cells were obtained through the same procedures as in the case of Example 1.

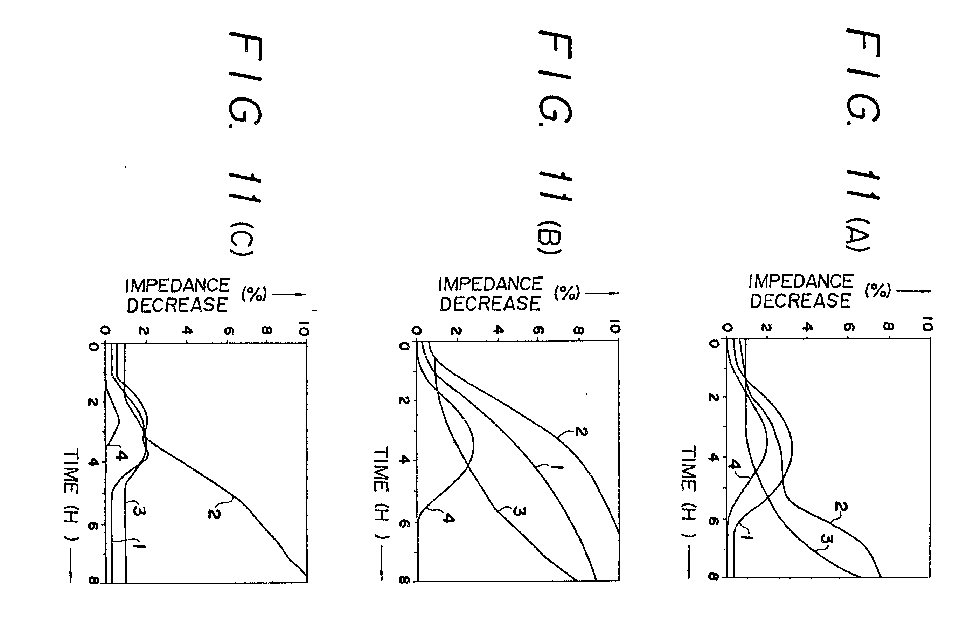

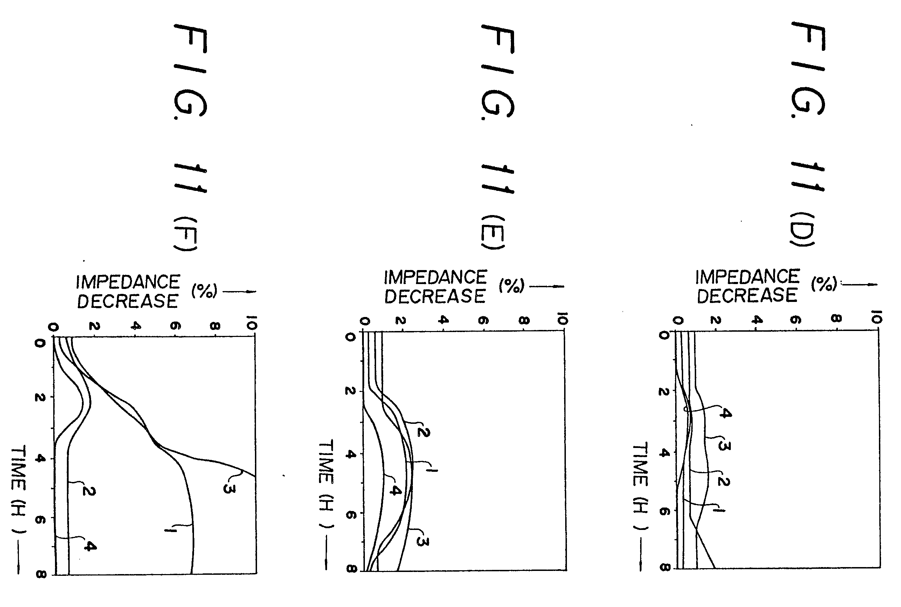

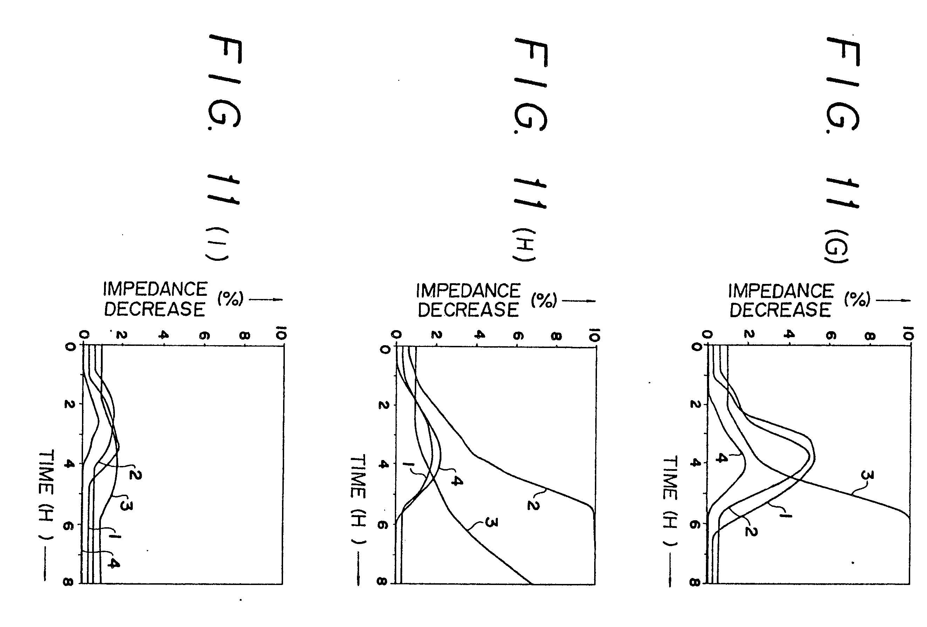

The results of impedance measurements made by using the instrument Orga 6 are graphically illustrated in Fig. 11(A) to Fig. 11(I). More specifically, Fig. 11(A) graphically illustrates the measurement results for *Escherichia coli*, Fig. 11(B) is for *Serratia marcescens*, Fig. 11(C) is for *Klebsiella pneumonia*, Fig. 11(D) is for *Klebsiella ozaenae*, Fig. 11(E) is for *Proteus vulgaris*, Fig. 11(F) is for *Enterobacter cloacae*, Fig. 11(G) is for *Proteus mirabilis*, Fig. 11(H) is for *Hafnia alvei*, and Fig. 11(I) is for *Citrobacter freundi*. In these figures, curves 1, 2 and 3 represent the impedance characteristic curves obtained for the uses of alginine, lysine and ornithine, respectively. A curve 4 corresponds to the case where no aminoacids are added.

It will be noted from these graphic illustrations that degree of utilization of aminoacids by each type of bacteria differs in dependence on the types of aminoacid. More particularly, the continuous decrease in impedance as observed from the associated impedance characteristic curve means that aminoacid is utilized by bacterium. On the other hand, when impedance turns to the increasing direction on the way, this means that aminoacids are not utilized by bacteria in concern. For example, in the case of *Escherichia coli*, ornithine and lysine are utilized or consumed, as can be seen from Fig. 11(A), while alginine is not utilized as will be seen from the profile of the associated curve which is similar to the one obtained in the case where no aminoacid is added. It is clear that the impedametrical method according to the invention allows decarboxylation reaction to be evaluated within six hours, which is very advantageous over the hitherto known agar plate method which requires an overnight cultivation. Such evaluation may be made by determining whether bacteria in concern are positive or negative for these three types of aminoacids and comparing with known data to thereby identify bacteria. In Table 2 recited below, utilizations of aminoacids by bacteria are represented by plus sign (positive) and minus sign (negative).

TABLE 2

Impedametrical evaluation of utilization of aminoacids by bacteria

| Aminoacids<br>Bacteria | Arginine | Lysine | Ornithine |
|---|---|---|---|
| Escherichia coli | − | + | + |
| Serratia marcescens | + | + | + |
| Klebsiella pneumonia | − | + | − |
| Klebsiella ozaenae | − | − | − |
| Proteus vulgaris | − | − | − |
| Enterobacter cloacae | + | − | + |
| Proteus mirabilis | − | − | + |
| Hafnia alvei | − | + | + |
| Citrobacter freundi | − | − | − |

It has been confirmed that the results listed in the Table 2 coincide with those obtained by using the commercially available enteric bacteria identifying kit "API 20E" stated above.

In this way, decarboxylation process can be detected as significant impedance changes when media which are prepared by adding glucose (5 g/l), aminoacids (each 10 g/l) and additionally disodium hydrogen phosphate (2.5 g/l) are used.

Next, method of identifying bacteria on the basis of the results as obtained will be described in concrete.

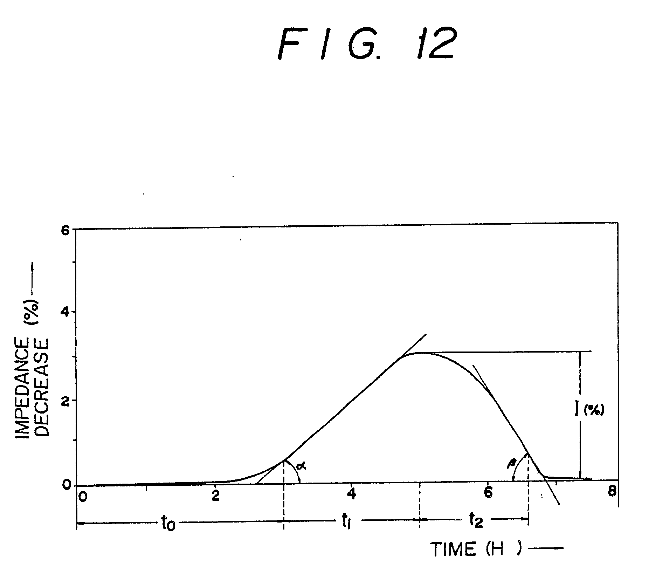

According to a first identification method, peculiar impedance changes brought about by bacteria in particular media are made use of. To this end, a curve representing impedance change by unknown bacterium is depicted in a manner illustrated in Fig. 12 and compared with the corresponding curves of known bacteria to thereby identify the unknown bacterium.

Parameters of an impedance characteristic curve involved in the comparative identification include rate $\alpha$ of impedance decrease for a unit time, rate $\beta$ of impedance increase for a unit time, a period $t_1$ during which impedance decreases, a period $t_2$ during which impedance increases and rate $I$ of impedance decrease immediately before impedance begins to increase, and, if required, a time span $t_o$ lying between the start of culture and the beginning of the impedance decrease. In principle, data obtained through actual measurement of various bacteria in the Example 1 are summarized in a table such as Table 3 incorporated hereinafter to be used as the known or reference data. Unknown bacteria are monitored under the same conditions and the resultant data are compared with the reference data to check whether the data obtained for the unknown bacteria coincide with the reference data within a permissible tolerance range. If the coincidence is found, it is estimated that the unknown bacteria are same types as those of reference.

### TABLE 3

| Types of bacteria \ Measured values | $\alpha(\%/h)$ | $\beta(\%/h)$ | $t_1(h)$ | $t_2(h)$ | $t_o(h)$ | $I(\%)$ |
|---|---|---|---|---|---|---|
| Escherichia coli | 1.1 | −1.1 | 2.5 | 2.2 | 1.5 | 2.1 |
| Serratia marcescens | 1.2 | −1.6 | 2.0 | 1.8 | 1.5 | 2.3 |
| Klebsiella pneumonia | 1.2 | −2.2 | 1.2 | 0.8 | 1.7 | 1.6 |
| Klebsiella ozaenae | 0.4 | −0.4 | 0.8 | 0.8 | 2.5 | 0.7 |
| Proteus vulgaris | 1.5 | −1.5 | 2.8 | 3.0 | 1.3 | 4.3 |
| Enterobacter cloacae | 1.4 | −2.3 | 1.0 | 0.8 | 1.1 | 1.5 |

According to a second identifying method, use is made of the results obtained in the Example 7. Utilizations of various carbohydrates by bacteria are detected in terms of impedance changes, whereby identification of bacteria is made by determining whether bacterium in concern is negative or positive.

According to a third identifying method, the results obtained in the Example 8 are used. Decarboxylations of various aminoacids by bacteria are detected in terms of impedance changes, whereby identification of bacteria is made by determining whether bacteria are positive or negative to aminoacids.

When the first, second and the third identifying methods are combined for making more comprehensive decision, identification of bacteria can be made with a much improved reliability and accuracy.

For practical applications, an electronic computer or data processing apparatus may be advantageously employed for carrying out the bacteria identifying methods described above in accordance with appropriately prepared programs in automated manner with a high accuracy without requiring a lot of time.

It should be mentioned here that in the figures described hereinbefore in conjunction with the Examples 1 to 8, the origin at which the impedance measurement is started is set slightly different among the individual samples.

As will be appreciated from the foregoing description, the invention has now provided methods and media which allow identification of bacteria to be made by measuring impedance change including decrease and increase in impedance which are brought about as bacteria achieve the growth. The method according to the invention can be applied to carbohydrate utilization test as well as decarboxylation test in which difficulty has heretofore been encountered in identifying micro-organisms by the conventional impedametrical method.

Since the invention can be applied to identification of microorganisms which are capable of utilizing carbohydrates, most of bacteria which are of interest in clinical areas can be identified by adopting the inventive method. Further, automated identification process can be realized on the basis of peculiar impedance change with a higher speed and higher accuracy as compared with the hitherto known identification which relies on change in color of indicators. Further, identification of bacteria on the basis of change in color requires a lot of time because growth of bacteria takes a relatively long period. In contrast thereto, the impedance change which is of interest to the present invention occurs within a relatively short time. Thus, the time required for establishing identity of bacteria can be significantly reduced.

# 0 036 274

**Claims**

1. A method of monitoring microorganisms comprising inoculating a growth medium with the microorganism and incubating the medium while measuring its changes in electrical impedance, characterised in that the medium contains both between 2.5 and 10 grams per litre of carbohydrate and between 1 and 10 grams per litre of sodium and/or potassium salts, and in that the incubation and impedance measurement are continued while the impedance first decreases, then passes through a minimum, and then again increases, to obtain an impedance-time curve characteristic of the microorganism.

2. A method of monitoring microorganisms according to claim 1, in which the rate of impedance decrease before reaching the minimum and the rate of impedance increase after passing the minimum are measured and are compared with known parameters to identify the microorganisms.

**Patentansprüche**

1. Verfahren zur Kontrolle von Mikroorganismen durch Beimpfung eines Wachstumsmediums mit dem Mikroorganismus und Bebrüten des Mediums unter Messen der Änderungen der elektrischen Impedanz, dadurch gekennzeichnet, daß das Medium 2,5 bis 10 g/l Kohlenhydrat und 1 bis 10 g/l Natrium- und/oder Kaliumsalze enthält und die Bebrütung und Impedanzmessung weitergeführt werden, während die Impedanz zuerst abnimmt, dann durch einen Minimalwert geht und wieder ansteigt, um eine für den Mikroorganismus charakteristische Impedanz-Zeit-Kurve zu erhalten.

2. Verfahren zur Kontrolle von Mikroorganismen nach Anspruch 1, bei welchem die Geschwindigkeit der Impedanzabnahme vor Erreichen des Minimalwertes und die Geschwindigkeit der Impedanzzunahme nach Durchgang durch den Minimalwert gemessen und zur Identifizierung der Mikroorganismen mit bekannten Parametern verglichen werden.

**Revendications**

1. Méthode pour contrôler des microorganismes comprenant l'inoculation d'un milieu de culture par le microorganisme et l'incubation du milieu tout en mesurant ses variations d'impédance électrique, caractérisée, en ce que le milieu contient à la fois entre 2,5 et 10 grammes par litre d'hydrate de carbone et entre 1 et 10 grammes par litre de sels de sodium et/ou de potassium, et en ce que l'incubation et la mesure de l'impédance sont poursuivies, l'impédance diminuant d'abord, puis passant par un minimum, et augmentant alors, de façon à obtenir une courbe impédance-temps caractéristique du microorganisme.

2. Méthode pour contrôler des microorganismes selon la revendication 1, dans laquelle le taux de diminution de l'impédance avant qu'elle atteigne le minimum et le taux d'augmentation de l'impédance après qu'elle ait dépassé le minimum sont mesurés et sont comparés à des paramètres connus de manière à identifier les microorganismes.

F I G. 1

F I G. 2

F I G. 3

1

F I G. 4 (A)

F I G. 4 (B)

F I G. 4 (C)

F I G. 4 (D)

F I G. 4 (E)

F I G. 4 (F)

3

F I G. 5 (A)

F I G. 5 (B)

F I G. 5 (C)

*F I G. 6*(A)

*F I G. 6*(B)

FIG. 6 (C)

FIG. 6 (D)

6

0 036 274

F I G. 7 (A)

F I G. 7 (B)

7

F I G. 7(C)

F I G. 7(D)

8

*F I G. 8*

*F I G. 9* (A)

*F I G. 9* (B)

9

F I G. 10 (A)  F I G. 10 (B)  F I G. 10 (C)

IMPEDANCE DECREASE (%) —

TIME (H) —

0 036 274

F I G. 10 (D)    F I G. 10 (E)    F I G. 10 (F)

0 036 274

F I G. 10 (G)  F I G. 10 (H)  F I G. 10 (I)

0 036 274

*F I G. 11* (A)

*F I G. 11* (B)

*F I G. 11* (C)

F I G. 11 (D)

F I G. 11 (E)

F I G. 11 (F)

FIG. 11 (G)

FIG. 11 (H)

FIG. 11 (I)

# F I G. 12